Europäisches Patentamt

European Patent Office

Office européen des brevets

(11) Numéro de publication: **0 216 668**
**B1**

(12) **FASCICULE DE BREVET EUROPEEN**

(45) Date de publication du fascicule du brevet:
21.03.90

(21) Numéro de dépôt: 86401839.5

(22) Date de dépôt: 19.08.86

(51) Int. Cl.⁴: **B01J 20/32**, B01J 20/26,
A61K 35/16, B01J 39/20,
C08F 261/04

(54) Support solide à base d'alcool polyvinylique capable d'absorber les lipoprotéines et son utilisation pour la séparation des lipoprotéines de basse densité présentes dans un liquide tel que du plasma sanguin.

(30) Priorité: 23.08.85 FR 8512666

(43) Date de publication de la demande:
01.04.87 Bulletin 87/14

(45) Mention de la délivrance du brevet:
21.03.90 Bulletin 90/12

(84) Etats contractants désignés:
BE CH DE FR GB IT LI NL SE

(56) Documents cités:
EP-A- 0 110 409
EP-A- 0 143 369
EP-A- 0 180 168
FR-A- 2 343 251
GB-A- 936 039

JOURNAL OF POLYMER SCIENCE: part A,
vol. 3, 1965, pages 3405-3412; B.S. BERNSTEIN et al.:
"Radiation crosslinking of nylon 66 and poly(vinyl alcohol)"

(73) Titulaire: Société anonyme: COMPAGNIE ORIS INDUSTRIE, 33, rue de la Fédération BP 510, F-75752 Paris Cédex(FR)

(72) Inventeur: Crassous, Geneviève, 27, rue Trousseau, F-75011 Paris(FR)
Inventeur: Gaussens, Gilbert, 11, rue Jean Brunet, F-92190 Meudon(FR)
Inventeur: Duval, Dominique, 11 rue Pierre Brossolette 333G, F-92400 Courbevoie(FR)
Inventeur: Nicaise, Maryvonne, 38, Avenue des Bois, F-78740 Saint Remy Les Chevreuse(FR)
Inventeur: Vergne, Gérard, 38, rue Albert Camus, F-92160 Antony(FR)
Inventeur: Hours, Sylvaine, 19, rue de la Félicité, F-75017 Paris(FR)

(74) Mandataire: Pottier, Pierre Société BREVATOME et al, 25, Rue de Ponthieu, F-75008 Paris(FR)

ACTORUM AG

EP 0 216 668 B1

## Description

La présente invention a pour objet un support solide capable d'adsorber les lipoprotéines, notamment les lipoprotéines de basse densité présentes dans un liquide tel que le plasma sanguin.

De façon plus précise, elle concerne des supports insolubles à base d'alcool polyvinylique, utilisables pour épurer sélectivement les lipoprotéines de basse densité du plasma sanguin par adsorption sélective.

Le taux des lipoprotéines de basse densité est anormalement élevé dans le sang de malades atteints d'hypercholestérolémie, ce qui peut entraîner des risques d'accidents cardiaques précoces par lésions vasculaires graves dues à l'accumulation de ces lipoprotéines. Aussi, des procédés permettant d'épurer le plasma sanguin de tels malades par adsorption des lipoprotéines de basse densité sur des supports solides présentent un grand intérêt car il n'existe actuellement aucune technique de ce genre pour effectuer cette épuration. En effet, on a utilisé jusqu'à présent des techniques de plasmaphérèse qui consistent à substituer en partie le plasma du malade par un plasma étranger ou par une solution de remplacement. Aussi, le développement de procédés d'épuration directe du plasma du malade fait l'objet de nombreuses recherches qui se justifient par l'intérêt d'éviter des injections de plasma étranger et la nécessité de maintenir les éléments plasmatiques constitutifs indispensables aux taux voulus.

A la suite de ces recherches, on a mis au point certains adsorbants pour l'épuration directe du plasma, comme il est décrit dans les documents EP-A 0 180 168, EP-A 0 143 369 et EP-A 0 110 409.

Dans le document EP-A 0 180 168, le support adsorbant est constitué par un polymère ayant des groupes hydroxy dont une partie au moins est convertie en groupes sulfate. Les polymères utilisés sont le polyacrylate, un mélange d'acétate de polyvinyle et d'alcool polyvinylique, et de la cellulose.

Dans le document EP-A 0 143 369, le support adsorbant comprend une suface sur laquelle est lié au moins un élément choisi parmi les groupes silanol et les polyanions synthétiques qui peuvent comporter des groupes fonctionnels tels que des groupes $SO_3H$. La surface peut être formée de gel de polymère vinylique tel que des copolymères réticulés.

Le document EP-A 0 110 409 illustre un support adsorbant constitué par un gel dur poreux sur lequel est fixé un ligand ayant une affinité pour les substances à séparer, le gel dur peut être en différents polymères synthétiques ou encore en produit inorganique.

La présente invention a pour objet un autre support solide adsorbant à base d'alcool polyvinylique.

La présente invention a précisément pour objet un support solide à base d'alcool polyvinylique, qui est capable de séparer sélectivement les lipoprotéines de basse densité présentes dans un liquide, et peut être utilisé pour réaliser ex-vivo l'épuration en lipoprotéines du plasma sanguin d'un malade atteint d'hypercholestérolémie.

Selon l'invention, le support solide capable d'adsorber les lipoprotéines, se caractérise en ce qu'il est constitué par un hydrogel d'alcool polyvinylique réticulé par irradiation avec au moins un monomère réticulant comportant au moins deux fonctions éthyléniques réactives sous rayonnements ionisants, ledit hydrogel d'alcool polyvinylique réticulé comportant de 70 à 95% en poids de chaînes dérivées de l'alcool polyvinylique et de 5 à 30% en poids de chaînes dérivés des monomères réticulants, et au moins une partie des groupes –OH de l'hydrogel d'alcool polyvinylique étant remplacée par des groupes $–OSO_3H$, les groupes $–OSO_3H$ représentant au moins 15% en poids du support.

Le fait d'utiliser un hydrogel d'alcool polyvinylique réticulé confère au support la propriété d'être insoluble dans la plupart des solvants, notamment dans l'eau et les solutions aqueuses, ce qui est indispensable lorsque l'on veut utiliser un tel support dans des procédés de séparation par adsorption en solution aqueuse. La réticulation permet aussi d'obtenir un produit insoluble qui résiste mécaniquement à l'action des solutions avec lesquelles il pourra être mis en contact, par exemple qui ne se désagrège pas dans une solution telle que du plasma sanguin. De plus, le fait de réaliser cette réticulation par irradiation au moyen de monomères réticulants permet de mieux contrôler le taux de réticulation et de faire en sorte que les groupes $–OSO_3H$ fixés sur l'alcool polyvinylique réticulé restent accessibles. En effet, la présence de monomères réticulants et l'irradiation permet d'obtenir un support insoluble ayant un gonflement dans les solutions aqueuses plus élevé que celui de supports en alcool polyvinylique réticulé sans adjonction de monomères réticulants. Ceci constitue un avantage important car il permet d'avoir une meilleure accessibilité aux groupes $-OSO_3H$ et d'obtenir de ce fait une meilleure adsorption des lipoprotéines de basse densité.

En effet, la présence des groupes $-OSO_3H$ confère au support une affinité spécifique pour les lipoprotéines qui présentent généralement la particularité de former des complexes stables avec des polyanions par l'intermédiaire de liaisons ioniques entre les groupements chargés négativement des polyanions tels que $SO^-_3$, $COO^-$ et $NHSO^-_3$ et les groupements positifs de la partie protéique des lipoprotéines tels que $NH^+_2$.

De préférence, le taux de réticulation de l'alcool polyvinylique n'est pas trop élevé pour que l'hydrogel conserve la propriété de gonfler dans l'eau, par exemple dans les liquides tels que le sérum physiologique. Cependant, il est nécessaire que le taux de réticulation soit suffisant pour obtenir les propriétés souhaitées d'insolubilité dans l'eau.

Ces deux résultats sont obtenus lorsque le support comprend :

- de 70 à 95 % en poids de chaînes dérivées de l'alcool polyvinylique, et

- de 5 à 30% en poids de chaînes dérivées de monomères réticulants comportant au moins deux fonctions éthyléniques réactives sous rayonnements ionisants.

Généralement, les monomères réticulants sont des monomères polyacryliques ou polyméthacryliques.

A titre d'exemple de tels monomères, on peut citer les polyacrylates et polyméthacrylates de polyol comme le diacrylate d'éthylène glycol, le diacrylate ou triacrylate de triéthylène glycol, le diacrylate de tétraéthylène glycol, le triacrylate de triméthylol propane, le diméthacrylate d'éthylène glycol, le diméthacrylate de triéthylène glycol, le diméthacrylate de tétraéthylène glycol, le triméthacrylate de triméthylol propane, le tétracrylate de pentaérythritol, le tétraméthacrylate de pentaérythritol et leurs mélanges.

Selon un mode préféré de réalisation de l'invention, les monomères réticulants sont du diacrylate de tétraéthylène glycol et du diacrylate de triéthylène glycol.

Généralement, les supports de l'invention sont utilisés sous la forme de poudres ayant une granulométrie de 50 à 100 µm, de préférence de 63 à 100 µm.

Dans les supports de l'invention, le taux de groupes -OSO$_3$H fixés sur le support détermine en particulier l'affinité du support vis-à-vis des lipoprotéines. Cette affinité augmente avec le nombre de groupes -OSO$_3$H et pour obtenir une affinité suffisante, il est nécessaire que le support comprenne au moins 15 % en poids de groupes -OSO$_3$H. Généralement de bons résultats sont obtenus lorsque le support comprend de 33 % à 45 % en poids de groupes -OSO$_3$H.

L'invention a également pour objet un procédé de préparation d'un support solide répondant aux caractéristiques précitées.

Ce procédé comprend les étapes suivantes :

a) - réticuler de l'alcool polyvinylique en soumettant à une irradiation au moyen de rayonnements ionisants un mélange comprenant de 70 à 95% en poids d'alcool polyvinylique et de 5 à 30% en poids d'au moins un monomère réticulant, en atmosphère exempte d'oxygène, et

b) soumettre l'alcool polyvinylique réticulé ainsi obtenu à un traitement de chlorosulfonation pour remplacer au moins une partie des groupes –OH de l'alcool polyvinylique par des groupes –OSO$_3$H de façon à obtenir un support comprenant au moins 15% en poids de groupes –OSO$_3$H.

Selon l'invention, on réalise ainsi la réticulation de l'alcool polyvinylique, d'une part, au moyen de rayonnements ionisants et, d'autre part, par réaction avec un monomère réticulant comportant au moins deux fonctions éthyléniques réactives sous rayonnements ionisants. Dans ce cas, on soumet à l'irradiation au moyen de rayonnements ionisants un mélange d'alcool polyvinylique et de monomères réticulants, en atmosphère exempte d'oxygène. Généralement, l'alcool polyvinylique est en solution aqueuse et l'on ajoute à cette solution les monomères réticulants qui peuvent être constitués en particulier par les monomères mentionnés précédemment.

Dans le procédé de l'invention, le choix de l'alcool polyvinylique utilisé comme produit de départ est également important car le poids moléculaire de celui-ci a un effet sur les résultats obtenus, en particulier sur le taux de gonflement dans l'eau du support et sur le taux d'adsorption des lipoprotéines.

De préférence, on utilise de l'alcool polyvinylique ayant en solution à 4% dans l'eau une viscosité de 3,5 à 5 mPa.s.

Pour la réticulation, les rayonnements ionisants susceptibles d'être utilisés sont par exemple les rayonnements ultraviolets, les rayonnements X, les rayonnements α, β ou γ et les faisceaux d'électrons accélérés.

On utilise avantageusement les rayonnements provenant d'une source de cobalt ou des faisceaux d'électrons. On peut aussi obtenir la réticulation par voie chimique exemple en utilisant des ions Ce$^{4+}$.

Dans le procédé de l'invention, les paramètres qui permettent d'agir sur le taux de réticulation sont :
- la concentration en alcool polyvinylique de la solution aqueuse,
- la dose d'irradiation,
- le débit de dose d'irradiation, et
- la quantité de monomères réticulants utilisée.

Lorsque l'alcool polyvinylique est irradié en solution aqueuse, la concentration en alcool polyvinylique de la solution est de préférence de 25% à 30% en poids.

Comme on l'a vu précédemment, la quantité de monomères réticulants utilisée représente au plus 30% en poids du mélange d'alcool polyvinylique et de monomères réticulants afin d'obtenir ensuite une affinité suffisante du support pour les lipoprotéines.

Les doses d'irradiation et le débit de doses d'irradiation utilisés sont choisis en fonction du taux de réticulation souhaité. Lorsque des monomères réticulants sont ajoutés à l'alcool polyvinylique, on tient compte également de la quantité de monomères réticulants utilisée pour choisir les doses d'irradiation et les débits de doses afin d'obtenir le taux de réticulation souhaité.

Généralement, lorsqu'on utilise des rayonnements γ, les doses totales d'irradiation sont de 8 à 16 Mrad et les débits de doses d'irradiation sont de 0,2 à 0,5 Mrad.h$^{-1}$.

La deuxième étape du procédé de l'invention, qui consiste en une chlorosulfonation de l'alcool polyvinylique réticulé, est généralement effectuée en milieu pyridine par réaction du polymère avec le complexe sulfate de pyridinium. On prépare ce complexe "in situ" par addition lente d'acide chlorosulfonique à de la pyridine maintenue à 0°C. On ajoute ensuite l'alcool polyvinylique réticulé et on poursuit la réaction à chaud, par exemple à 70°C, sous agitation.

La durée de réaction est choisie en fonction de la quantité de groupes -OSO₃H que l'on veut fixer sur l'alcool polyvinylique réticulé.

Après cette réaction, on sépare l'hydrogel de la solution et on le soumet à différents lavages, par exemple par des mélanges méthanol-soude. On sèche ensuite l'hydrogel obtenu dans une étuve, puis on le broie, on le tamise à la granulométrie voulue et on le conserve à l'état sec. Avant d'être utilisée comme support adsorbant des lipoprotéines, la poudre de polymère est gonflée au préalable dans du sérum physiologique.

La présente invention a également pour objet un procédé de séparation des lipoprotéines de basse densité présentes dans un liquide.

Ce procécé consiste à mettre en contact le liquide avec le support solide de l'invention, et à séparer ensuite le liquide du support sur lequel ont été adsorbées les lipoprotéines de basse densité.

Le liquide peut être du sang ou du plasma sanguin.

D'autres caractéristiques et avantages de l'invention apparaîtront mieux à la lecture de la description qui suit, donnée bien entendu à titre illustratif et non limitatif en référence au dessin annexé sur lequel :

- la figure 1 est un diagramme représentant l'influence du taux de monomères réticulants sur le taux de gonflement des supports dans du sérum physiologique,
- la figure 2 est un diagramme représentant l'influence de la viscosité de l'alcool polyvinylique sur le pourcentage d'épuration en cholestérol total (courbe 1) et sur le taux de gonflement dans du sérum physiologique (courbe 2),
- la figure 3 est un diagramme représentant l'influence du taux de monomères réticulants sur le pourcentage d'épuration en cholestérol total pour différentes doses d'irradiation, et
- la figure 4 représente schématiquement un type d'épurateur plasmatique pour mettre en oeuvre le procédé de l'invention.

Les exemples suivants sont donnés à titre d'illustration de l'invention. Dans tous ces exemples, les supports sont constitués par des hydrogels d'alcool polyvinylique (PVA) réticulé sous irradiation avec un mélange de diacrylate de tétraéthylène glycol et de diacrylate de triéthylène glycol (DIATEG).

EXEMPLES 1 à 5

Dans ces exemples, on part d'alcool polyvinylique ayant en solution aqueuse à 4% une viscosité de 3,5 à 5 mPa.s et on réalise la réticulation de cet alcool polyvinylique en soumettant une solution aqueuse contenant 30% en poids d'alcool polyvinylique à laquelle on ajoute du DIATEG (mélange de diacrylate de triéthylène glycol et de diacrylate de tétraéthylène glycol) pour que le mélange DIATEG/ALCOOL polyvinylique contienne de 10 à 40% en poids de DIATEG et 60 à 90% en poids de PVA. On soumet à une irradiation sous faisceaux d'électrons de façon que la dose totale d'irradiation soit de 12 Mrad avec un débit de dose moyen de 134 Mrad.h⁻¹. Après cette étape, on soumet l'alcool polyvinylique réticulé à une chlorosulfonation en mettant en contact l'alcool polyvinylique réticulé avec une solution d'acide chlorosulfonique et de pyridine à 12% d'acide chlorosulfonique, pendant 3 heures, 70° C, sous agitation. On lave ensuite l'hydrogel par des mélanges de méthanol et de soude, on le sèche dans une étuve, on le broie et on le tamise afin de recueillir la poudre ayant une granulométrie de 63 à 80 µm, puis on la conserve à l'état sec. On détermine alors le taux de groupes -OSO₃H fixés sur le support à partir du taux de soufre déterminé par analyse élémentaire (AE).

On contrôle ensuite les propriétés du support ainsi obtenu pour la séparation des lipoprotéines du plasma sanguin. Dans ce but, on immerge tout d'abord la poudre dans du sérum physiologique pour la gonfler, puis on réalise les essais, d'adsorption des lipoprotéines. Pour ces essais on utilise une cuve fermée, dans laquelle on introduit 1 ml du support en poudre et 3 ml du plasma à épurer contenant les lipoprotéines. On maintient l'ensemble sous agitation rotative permanente et on laisse incuber pendant 30 min à la température ambiante.

En fin de réaction, on sépare le plasma du support par décantation ou centrifugation et on détermine les taux de lipoprotéines : cholestérol total $C_T$, et lipoprotéines de haute densité $C_{HDL}$ du plasma. On a déterminé également les taux de lipoprotéines avant la réalisation du traitement d'épuration.

On en déduit ainsi le taux d'épuration obtenu. Pour déterminer les différents taux, on opère de la façon suivante : on mesure le taux de cholestérol total ($C_T$) par dosage colorimétrique après hydrolyse enzymatique en utilisant la trousse Sigma et la méthode décrite dans Allain. CA, Poon. LS, Cran C.S.G, Richmond W, Fu PC., Clin. Chem, 20, p. 470 (74) Enzymatic determination of total serum cholesterol ; on mesure le taux de lipoprotéines de haute densité ($C_{HDL}$) par dosage colorimétrique dans le surnageant après précipitation sélective des lipoprotéines de basse densité et des lipoprotéines de très basse densité par le phosphotungstate de sodium en présence de $MgCl_2$. On détermine également le taux de triglycérides ($T_G$) du plasma par dosage en ultraviolet après hydrolyse enzymatique en utilisant la trousse SIGMA. Après avoir effectué ces dosages, on peut en déduire le taux de lipoprotéines de basse densité $C_{LDL}$ en utilisant la formule :

$$C_{LDL} = C_T - C_{HDL} - \frac{T_G}{5}$$

Après avoir effectué ces mesures, on détermine la capacité d'épuration du support qui correspond à la quantité de cholestérol total $C_T$ (en mg) fixée par ml de support gonflé dans le sérum physiologique. Le taux d'épuration est donné par le pourcentage de $C_T$ fixé qui est déterminé à partir des concentrations plasmatiques en $C_T$ avant et après épuration. On contrôle le taux d'adsorption des lipoprotéines de haute densité $C_{HDL}$ adsorbées. On contrôle également le taux des lipoprotéines de basse densité $C_{LDL}$ adsorbées ainsi que le taux de triglycérides ($T_G$). Les résultats obtenus sont donnés dans le tableau 1 où l'on a reporté également les conditions de fabrication du support. Au vu de ce tableau, on constate que la capacité d'épuration diminue lorsque la proportion d'agent réticulant augmente. Par ailleurs, on a contrôlé que l'adsorption des lipoprotéines de haute densité ne dépasse jamais 20% de leur valeur initiale dans le plasma. Donc les supports présentent une bonne sélectivité pour les lipoprotéines de basse densité.

EXEMPLES 6 à 8

Dans ces exemples, on utilise le même mode opératoire que dans l'exemple 1 pour fabriquer et tester des supports préparés à partir du même alcool polyvinylique et de DIATEG, mais dans ce cas, on fait varier la dose totale d'irradiation en utilisant une teneur en DIATEG du mélange PVA-DIATEG de 20%. Après le traitement de chlorosulfonation, on recueille la poudre ayant une granulométrie de 63 à 100 µm, on détermine sa teneur en groupes -OSO₃H et on la soumet aux essais d'adsorption des lipoprotéines dans les mêmes conditions que celles des exemples 1 à 5. Les résultats obtenus sont donnés dans le tableau 1.

Au vu de ces résultats, on constate que la capacité d'épuration diminue lorsque la dose d'irradiation augmente, c'est-à-dire lorsque le taux de réticulation augmente.

EXEMPLES 9 à 15

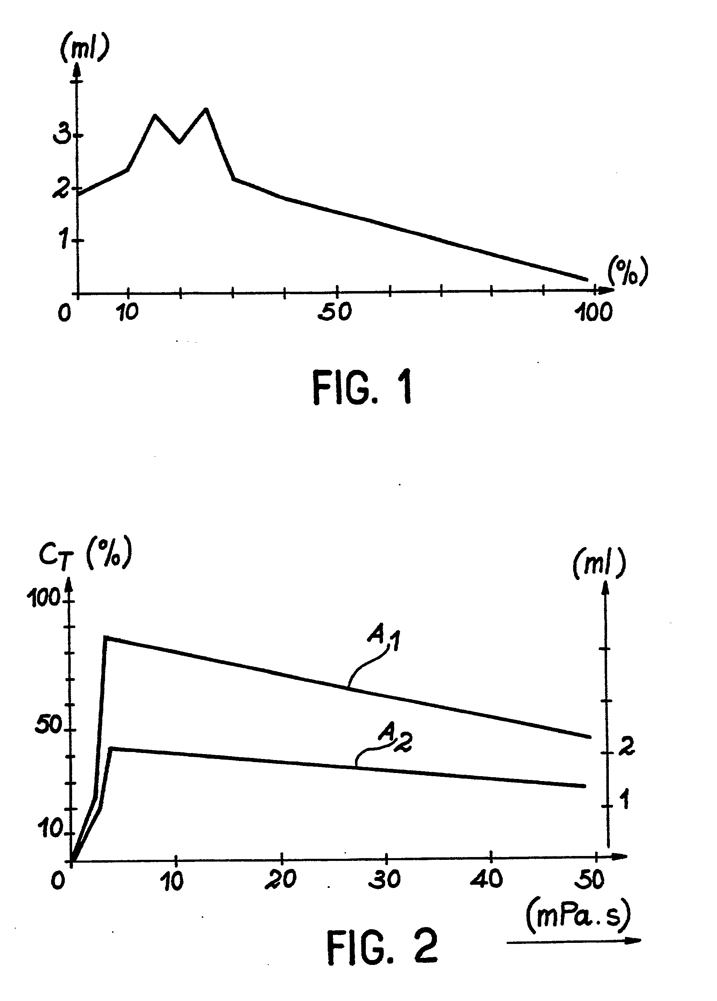

Dans ces exemples, on utilise le même alcool polyvinylique et le même mode opératoire que dans l'exemple 1 pour préparer l'alcool polyvinylique réticulé et pour réaliser le traitement de chlorosulfonation mais on fait varier la teneur en DIATEG du mélange PVA-DIATEG de 0 à 40% et l'on applique une dose d'irradiation de 10 Mrad dans le cas où il n'y a pas de DIATEG et de 14 Mrad dans le cas où l'on réalise la réticulation avec du DIATEG. On mesure ensuite la teneur en groupes -OSO₃H et la capacité d'épuration du support pour les lipoprotéines comme dans l'exemple 1 et l'on détermine également le taux de gonflement des supports dans le sérum physiologique (SØ). Les résultats obtenus sont donnés dans le tableau 2 qui suit et sur la figure 1 qui représente les variations du taux de gonflement (en ml) de 100 mg de support en fonction de la teneur en DIATEG (% en poids).

La figure 1 montre que le gonflement du support est plus important lorsque la teneur en DIATEG est de 5 à 30%. Cette amélioration du gonflement est une propriété très intéressante des supports de l'invention, car elle permet d'obtenir une meilleure accessibilité des groupes -OSO₃H utilisés pour l'épuration des lipoprotéines de basse densité. On constate au vu de cette figure que cette amélioration est obtenue grâce à l'addition de monomères réticulants.

Les résultats du tableau 2 montrent également que les taux d'épuration sont meilleurs lorsque la proportion de DIATEG ne dépasse pas 25% et qu'il en est de même des taux de gonflement.

EXEMPLES 16 à 25

Dans ces exemples, on étudie l'influence de la granulométrie des particules de support sur la capacité d'épuration. Les supports sont préparés comme dans l'exemple 1 en partant du même PVA, mais en utilisant 15, 20 ou 25% en poids de DIATEG et en réalisant l'irradiation au moyen des rayonnements γ d'une source de cobalt 60 avec une dose totale de 14 Mrad et un débit de dose de 0,23 Mrad.h⁻¹. Après le traitement de chlorosulfonation qui est réalisé dans les mêmes conditions que celles de l'exemple 1, on lave, on sèche, on broie et on tamise l'hydrogel d'alcool polyvinylique ainsi obtenu. On isole des fractions ayant des granulométries allant de 50 à 63 de 63 à 80, de 80 à 100 et de 100 à 200 µm, puis on utilise chaque fraction de granulométrie donnée pour réaliser les essais d'adsorption des lipoprotéines en utilisant les mêmes conditions d'essais que celles de l'exemple 1. Les résultats obtenus sont donnés dans le tableau 3.

Au vu de ces résultats, on constate que la capacité d'épuration est sensiblement meilleure lorsque la granulométrie du support est de 50 à 80 µm.

EXEMPLES 26 à 29

Dans ces exemples, on étudie l'influence du poids moléculaire de l'alcool polyvinylique de départ sur le résultat obtenu.

.Les alcools polyvinyliques de départ sont des produits commerciaux dont la viscosité en solution aqueuse à 4% peut varier de 2,6 à 48 mPa.s. On réticule ces supports par irradiation au moyen des rayonnements $\gamma$ d'une source de cobalt 60 avec 25% de DIATEG, une dose totale d'irradiation de 14 Mrad et un débit de dose de 0,23 Mrad.h$^{-1}$. On soumet ensuite les hydrogels réticulés ainsi obtenus à un traitement de chlorosulfonation réalisé dans les mêmes conditions que celles de l'exemple 1. On isole la fraction ayant une granulométrie de 60 à 100 $\mu$m et on détermine ensuite le taux de gonflement (en ml) de 100 mg du support dans du sérum physiologique et la capacité d'épuration des lipoprotéines. Les résultats obtenus sont donnés dans le tableau 4 et sur les courbes A$_1$ et A$_2$ de la figure 2 qui représentent respectivement les variations de la capacité d'épuration C$_T$ (en %) et du gonflement (en ml) de 100 mg de support dans du sérum physiologique en fonction de la viscosité (en mPa.s) de l'alcool polyvinylique de départ.

Au vu de ces résultats, on constate que le taux de gonflement et la capacité d'épuration sont meilleurs lorsque la viscosité de l'alcool polyvinylique de départ varie de 3,5 à 5 mPa.s.

EXEMPLE 30

Dans cet exemple, on reprend le mode opératoire de l'exemple 1 et on étudie l'influence de la teneur en DIATEG (en %) et des conditions d'irradiation sur la capacité d'épuration C$_T$ (en %) des supports solides obtenus. Les résultats sont donnés sur la figure 3 où les courbes A$_3$ à A$_5$ se réfèrent respectivement à des essais effectués sur des supports en poudre ayant une granulométrie de 50 à 63 $\mu$m pour la courbe A$_3$, de 63 à 80 $\mu$m pour la courbe A$_4$ et de 100 à 800 $\mu$m pour la courbe A$_5$, l'irradiation étant réalisée avec une dose totale de 14 Mrad au moyen de rayonnements $\gamma$.

La courbe A$_6$ se réfère à des essais réalisés sur un support en poudre ayant une granulométrie de 63 à 800 $\mu$m irradié au moyen d'un faisceau d'électrons à une dose totale de 12 Mrad.

Au vu de cette figure, on constate que les meilleurs résultats sont obtenus lorsque la quantité de DIATEG est inférieure à 25%. Par ailleurs, l'irradiation au moyen des rayonnements $\gamma$ permet d'obtenir de meilleurs résultats que l'irradiation au moyen d'un faisceau d'électrons.

EXEMPLE 31

Dans cet exemple, on reprend le mode opératoire de l'exemple 1 pour fabriquer un support à partir du même alcool polyvinylique, mais en utilisant comme monomère réticulant du tétraméthacrylate de pentacrythritol (TMPTA) la teneur en TMPTA du mélange PVA-TMPTA étant de 8,5% en poids. On réalise l'irradiation au moyen des rayonnements $\gamma$ provenant d'une source de cobalt 60 avec une dose totale d'irradiation de 14 Mrad.

Après le traitement de chlorosulfonation réalisé dans les mêmes conditions que celles de l'exemple 1, on recueille la fraction ayant une granulométrie de 63 à 100 $\mu$m et on détermine comme précédemment le taux de gonflement de 100 mg de support dans du sérum physiologique et la capacité d'épuration du support pour les lipoprotéines. Les résultats obtenus sont donnés dans le tableau 5 qui suit.

EXEMPLE 32

Dans cet exemple, on reprend le mode opératoire de l'exemple 1 pour fabriquer un support à partir du même alcool polyvinylique, mais en utilisant comme monomère réticulant du tétracrylate de pentaérythritol (TTPE), la teneur en TTPE du mélange PVA-TTPE étant de 8,5% en poids. On réalise l'irradiation au moyen des rayonnements $\gamma$ provenant d'une source de cobalt 60 avec une dose totale d'irradiation de 14 Mrad.

Après le traitement de chlorosulfonation réalisé dans les mêmes conditions que celles de l'exemple 1, on recueille la fraction ayant une granulométrie de 63 à 100 $\mu$m et on détermine comme précédemment le taux de gonflement dans du sérum physiologique de 100 g de support et la capacité d'épuration du support pour les lipoprotéines. Les résultats obtenus sont donnés dans le tableau 6 qui suit.

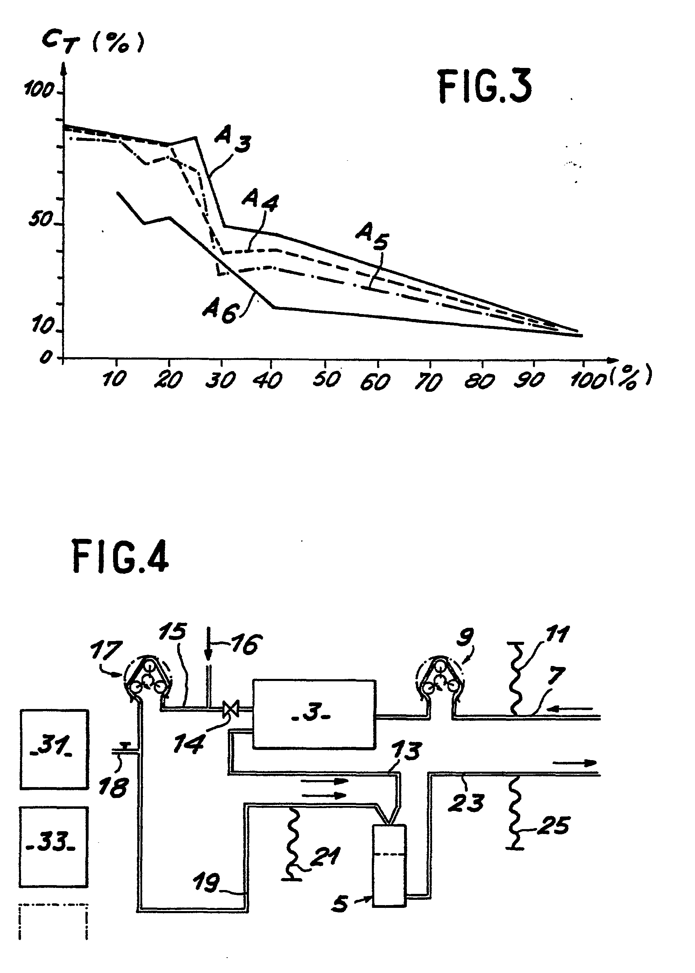

Les supports de l'invention peuvent être utilisés pour l'épuration du sang total ou du plasma sanguin dans l'épurateur représenté sur la figure 4.

Sur la figure 4, on voit que l'épurateur comprend un séparateur de cellules 3 pour isoler le plasma sanguin du sang à épurer et un collecteur 5 dans lequel sont introduits, d'une part, les cellules séparées (en 3) du sang à traiter et, d'autre part, le plasma épuré.

Dans cette installation, une première conduite 7 munie d'une pompe 9 et d'un moyen de mesure de la pression 11 sert à introduire le sang provenant du patient dans le séparateur de cellules 3. Dans ce séparateur, les cellules sont évacuées par la conduite 13 vers le collecteur 5 tandis que le plasma est dirigé dans la conduite 15 munie d'une pompe 17. Celui-ci est ensuite extrait du circuit par la vanne de soutirage 18 puis introduit dans l'une des poche 31 ou 33 contenant le support adsorbant de l'invention pour y être épuré. Après épuration, on réintroduit le plasma épuré en amont de la pompe 17 par la conduite 16 alors que la vanne 14 est fermée. Celui-ci est alors évacué par la conduite 19 munie d'un moyen de mesure de pression 21 dans le collecteur 5 qui constitue, de plus, un système de sécurité pour éviter la présence de bulles dans le sang ainsi reconstitué. Ce dernier est alors évacué par la conduite 23 munie du moyen de mesure de pression 25 dans le système circulatoire du patient.

TABLEAU 1

| EXEMPLES | % en poids du DIATEG dans le mélange PVA-DIATEG | Dose d'irra-diation (Mrad) | Granulométrie (µm) | % en poids de Soufre (AE) |
|---|---|---|---|---|
| 1 | 10 | 12 | 63 - 80 | 13,8 |
| 2 | 15 | 12 | 63 - 80 | 13 |
| 3 | 20 | 12 | 63 - 80 | 15 |
| 4 | 25 | 12 | 63 - 80 | 15 |
| 5 | 40 | 12 | 63 - 80 | 11,5 |
| 6 | 20 | 8,8 | 63 -100 | 14,5 |
| 7 | 20 | 12 | 63 -100 | 15 |
| 8 | 20 | 16 | 63 -100 | 15 |

## TABLEAU 1 (Suite)

| EXEMPLES | CAPACITE D'EPURATION | | | | | | | |
|---|---|---|---|---|---|---|---|---|
| | $C_T$ (mg/ml de support) | $C_T$ (%) | $C_{HDL}$ (mg/ml de support) | $C_{HDL}$ (%) | $T_G$ (mg/ml de support) | $T_G$ (%) | $C_{LDL}$ (mg/ml de support) | $C_{LDL}$ (%) |
| 1 | 5,5 | 64 | 0,093 | 20 | 0,36 | 25,5 | 5,33 | 68 |
| 2 | 4,4 | 50 | 0,126 | 27 | 0,27 | 19 | 4,22 | 52 |
| 3 | 4 | 54 | 0,093 | 20 | 0,18 | 13 | 3,87 | 58 |
| 4 | 5 | 58 | 0,135 | 29 | 0,60 | 42,5 | 4,75 | 60 |
| 5 | 1,9 | 21 | 0,135 | 29 | 0,21 | 15 | 1,72 | 21 |
| 6 | 5 | 70 | 0,069 | 15 | 0,55 | 39 | 4,82 | 75 |
| 7 | 4 | 54 | 0,093 | 20 | 0,18 | 13 | 3,87 | 58 |
| 8 | 1 | 18 | 0,075 | 16 | 0,20 | 14 | 0,90 | 18 |

EP 0 216 668 B1

## TABLEAU 2

| EXEMPLES | % en poids du DIATEG dans le mélange PVA-DIATEG | Dose d'irra-diation (Mrad) | Gonflement dans sérum physiologique (en ml) | % en poids Soufre (AE) |
|---|---|---|---|---|
| 9 | 0 | 10 | 1,9 | 12 |
| 10 | 10 | 14 | 2,3 | 11,5 |
| 11 | 15 | 14 | 3,3 | 13,5 |
| 12 | 20 | 14 | 2,9 | 13,7 |
| 13 | 25 | 14 | 3,4 | 13 |
| 14 | 30 | 14 | 2,2 | 13 |
| 15 | 40 | 14 | 1,8 | 12 |

TABLEAU 2 (suite)

| EXEMPLES | CAPACITE D'EPURATION | | | | | | | |
|---|---|---|---|---|---|---|---|---|
| | $C_T$ | | $C_{HDL}$ | | $T_G$ | | $C_{LDL}$ | |
| | mg/ml de support | (%) | mg/ml de support | (%) | mg/ml de support | (%) | mg/ml de support | (%) |
| 9 | 7,5 | 85 | 0,105 | 22 | 0,108 | 8 | 7,4 | 91 |
| 10 | 7,2 | 83 | 0,135 | 29 | 0,48 | 34 | 7 | 88 |
| 11 | 7 | 80 | 0,120 | 26 | 0,63 | 45 | 6,8 | 84 |
| 12 | 7 | 80 | 0,105 | 22 | 0,55 | 39 | 6,8 | 84 |
| 13 | 7 | 80 | 0,174 | 37 | 0,75 | 53 | 6,7 | 83 |
| 14 | 3,5 | 39 | 0,186 | 40 | 0,48 | 34 | 3,22 | 39 |
| 15 | 4,5 | 12 | 0,195 | 42 | 0,51 | 36 | 4,20 | 11 |

TABLEAU 3

| EXEMPLES | % en poids du DIATEG dans le mélange PVA-DIATEG | Dose d'irradiation (Mrad) | Granulométrie (µm) |
|---|---|---|---|
| 16 | 15 | 14 | 80-100 |
| 17 | | | 63- 80 |
| 18 | | | 50- 63 |
| 19 | 20 | 14 | 100-200 |
| 20 | | | 80-100 |
| 21 | | | 63- 80 |
| 22 | | | 50- 63 |
| 23 | 25 | 14 | 80-100 |
| 24 | | | 63- 80 |
| 25 | | | 50- 63 |

EP 0 216 668 B1

## TABLEAU 3 (suite)

| EXEMPLES | CAPACITE D'EPURATION | | | |
|---|---|---|---|---|
| | $C_T$ (mg/ml de support | $C_T$ (%) | $C_{HDL}$ (mg/ml de support | $C_{HDL}$ (%) |
| 16 | 6,5 | 75 | 0,135 | 24 |
| 17 | 6,9 | 80 | 0,09 | 16 |
| 18 | 7 | 82 | 0,10 | 19 |
| 19 | 5 | 48 | 0,06 | 11 |
| 20 | 6,6 | 77 | 0,09 | 16 |
| 21 | 7 | 80 | 0,075 | 13,5 |
| 22 | 7 | 80 | 0,10 | 19 |
| 23 | 6 | 71 | 0,09 | 16 |
| 24 | 7 | 80 | 0,075 | 13,5 |
| 25 | 7,4 | 84 | 0,075 | 13,5 |

## TABLEAU 4

| EXEMPLES | PVA | Viscosité en solution à 4% (mPa.s) | taux de gonflement (ml) | Capacité d'épuration $C_T$ (%) |
|---|---|---|---|---|
| 26 | Mowiol 3-83 | 2,6 | 1,1 | 25 |
| 27 | Rhodoviol 4-125 | 3,5-5 | 2,2 | 86 |
| 28 | Polyviol*G04-20 | 4 | 2,1 | 87 |
| 29 | Polyviol*G48-20 | 48 | 1,4 | 47 |

* Polyviol G04-20 : degré de polymérisation en nombre = 400

* Polyviol G48-20 : degré de polymérisation en nombre = 2000

EP 0 216 668 B1

## TABLEAU 5

| Exemple | % en poids du TMPTA dans le mélange PVA-TMPTA | dose d'irradiation (MRad) | granulométrie ($\mu$ m) | gonflement dans le sérum physiologique (en ml) |
|---------|------|------|------|------|
| 31 | 8,5 | 14 | 63-100 | 3,1 |

| EXEMPLE | CAPACITE D'EPURATION | | | | | | | |
|---------|------|------|------|------|------|------|------|------|
| | $C_T$ (mg/ml de support | $C_T$ (%) | $C_{HDL}$ (mg/ml de support | $C_{HDL}$ (%) | $T_G$ (mg/ml de support | $T_G$ (%) | $C_{LDL}$ mg/ml de support | $C_{LDL}$ (%) |
| 31 | 2 | 20 | 0,18 | 33 | 1,44 | 35 | 2 | 19 |

EP 0 216 668 B1

EP 0 216 668 B1

**TABLEAU 6**

| Exemple | % en poids du TTPE dans le mélange PVA-TTPE | dose d'irradiation ( Mrad) | granulométrie ($\mu$ m) | gonflement dans le sérum physiologique (en ml) | Capacité d'épuration | |
|---|---|---|---|---|---|---|
| | | | | | $C_T$ mg/ml de support | $C_T$(%) |
| 32 | 8,5 | 14 | 63-100 | 3,7 | 2,5 | 25 |

## Revendications

1. Support solide, capable d'absorber les lipoprotéins, caractérisé en ce qu'il est constitué par un hydrogel d'alcool polyvinylique réticulé par irradiation avec au moins un monomère réticulant comportant au moins deux fonctions éthyléniques réactives sous rayonnements ionisants, ledit hydrogel d'alcool polyvinylique réticulé comportant de 70 à 95% en poids de chaînes dérivées de l'alcool polyvinylique et de 5 à 30% en poids de chaînes dérivées des monomères réticulants, et au moins une partie des groupes –OH de l'hydrogel d'alcool polyvinylique étant remplacée par des groupes –OSO$_3$H, les groupes –OSO$_3$H représentant au moins 15% en poids du support.

2. Support selon la revendication 1, caractérisé en ce que les monomères réticulants sont choisis parmi le diacrylate d'éthylène glycol, le diacrylate de triéthylène glycol, le diacrylate ou triacrylate de tétraéthylène glycol, le triacrylate de triméthylol propane, le diméthacrylate d'éthylène glycol, le diméthacrylate de triéthylène glycol, le diméthacrylate de tétraéthylène glycol, le triméthacrylate de triméthylol propane, le tétracrylate de pentaérythritol le tétraméthacrylate pentaérythritol et leurs mélanges.

3. Support solide selon la revendication 1, caractérisé en ce que les monomères réticulants sont le diacrylate de triéthylène glycol et le diacrylate de tétraéthylène glycol.

4. Support solide selon l'une quelconque des revendications 1 à 3, caractérisé en ce qu'il est sous la forme d'une poudre ayant une granulométrie de 50 à 100 µm.

5. Procédé de préparation d'un support solide selon l'une quelconque des revendications 1 à 4, caractérisé en ce qu'il comprend les étapes suivantes:

   a) réticuler de l'alcool polyvinylique en soumettant à une irradiation au moyen de rayonnements ionisants un mélange comprenant de 70 à 95% en poids d'alcool polyvinylique et de 5 à 30% en poids d'au moins un monomère réticulant, en atmosphère exempte d'oxygène, et

   b) soumettre l'alcool polyvinylique réticulé ainsi obtenu à un traitement de chlorosulfonation pour remplacer au moins une partie des groupes –OH de l'alcool polyvinylique par des groupes –OSO$_3$H de façon à obtenir un support comprenant au moins 15% de groupes –OSO$_3$H.

6. Procédé selon la revendication 5, caractérisé en ce que lors de l'étape a), l'alcool polyvinylique est en solution aqueuse.

7. Procédé selon l'une quelconque des revendications 5 et 6, caractérisé en ce que la concentration en alcool polyvinylique de la solution aqueuse est de 25 à 30% en poids.

8. Procédé selon l'une quelconque des revendications 5 à 7, caractérisé en ce que l'alcool polyvinylique de départ en solution aqueuse à 4% une viscosité de 3,5 à 5 mPa.s.

9. Procédé selon l'une quelconque des revendications 5 à 8, caractérisé en ce que l'irradiation étant effectuée au moyen de raynonnements γ, la dose totale d'irradiation est de 8 à 16 Mrad.

10. Procédé selon l'une quelconque des revendications 5 à 9, caractérisé en ce que le traitement de chlorosulfonation consiste à préparer un complexe d'acide chlorosulfonique et de pyridine, puis à mettre en contact ce complexe avec l'alcool polyvinylique réticulé.

11. Procédé de séparation des lipoprotéines présentes dans un liquide, caractérisé en ce qu'il consiste:

   – à mettre en contact ledit liquide avec un support solide selon l'une quelconque des revendications 1 à 4, et

   – à séparer ensuite le liquide du support sur lequel ont été adsorbées les lipoprotéines.

12. Procédé selon la revendication 11, caractérisé en ce que le liquide est du sang.

13. Procédé selon la revendication 11, caractérisé en ce que le liquide est du plasma sanguin.

## Claims

1. Solid support able to adsorb lipoproteins, characterized in that it is constituted by a polyvinyl alcohol hydrogel crosslinked by irradiation with at least one crosslinking monomer having at least two reactive ethylene functions under ionizing radiation, said crosslinked polyvinyl alcohol hydrogel having 70 to 95% by weight of chains derived from polyvinyl alcohol and 5 to 30% by weight of chains derived from crosslinking monomers and at least part of the –OH groups of the polyvinyl alcohol hydrogel are replaced by –OSO$_3$H groups, the latter representing at least 15% by weight of the support.

2. Support according to claim 1, characterized in that the crosslinking monomers are chosen from among ethylene glycol diacrylate, triethylene glycol diacrylate, tetraethylene glycol diacrylate or triacrylate, trimethylol propane triacrylate, ethylene glycol dimethacrylate, triethylene glycol dimethacrylate, tetraethylene glycol dimethacrylate, trimethylol propane trimethacrylate, pentaerythritol tetraacrylate, pentaerythritol tetramethacrylate and mixtures thereof.

3. Solid support according to claim 1, characterized in that the crosslinking monomers are triethylene glycol diacrylate and tetraethylene glycol diacrylate.

4. Solid support according to any one of the claims 1 to 3, characterized in that it is in the form of a powder having a grain size of 50 to 100 µm.

5. Process for the preparation of a solid support according to any one of the claims 1 to 4, characterized in that it comprises the following stages:

a) crosslinking the polyvinyl alcohol by irradiating by means of ionizing rays a mixture of 70 to 95% by weight polyvinyl alcohol and 5 to 30% by weight of crosslinking monomers, in an oxygen-free atmosphere and

b) subjecting the thus obtained crosslinked polyvinyl alcohol to a chlorosulphonation treatment to replace at least part of the $-OH$ groups of the polyvinyl alcohol by $-OSO_3H$ groups, so as to obtain a support comprising at least 15% by weight of $-OSO_3H$ groups.

6. Process according to claim 5, characterized in that during stage a), the polyvinyl alcohol is in aqueous solution.

7. Process according to either of the claims 5 and 6, characterized in that the polyvinyl alcohol concentration of the aqueous solution is 25 to 30% by weight.

8. Process according to any one of the claims 5 to 7, characterized in that the starting polyvinyl alcohol in a 4% aqueous solution has a viscosity of 3.5 to 5 mPa•s.

9. Process according to any one of the claims 5 to 8, characterized in that irradiation is carried out by means of gamma rays, the total radiation dose being 8 to 16 Mrad.

10. Process according to any one of the claims 5 to 9, characterized in that the chlorosulphonation treatment consists of preparing a complex of chlorosulphonic acid and pyridine, then contacting said complex with the crosslinked polyvinyl alcohol.

11. Process for the separation of lipoproteins present in a liquid, characterized in that it comprises contacting said liquid with a solid support according to any one of the claims 1 to 4 and then separating the liquid from the support on which the lipoproteins have been adsorbed.

12. Process according to claim 11, characterized in that the liquid is blood.

13. Process according to claim 11, characterized in that the liquid is blood plasma.


**Patentansprüche**

1. Fester Träger, der Lipoproteine adsorbieren kann, dadurch gekennzeichnet, daß er besteht aus einem durch Bestrahlung vernetzten Polyvinylalkohol-Hydrogel mit mindestens einem vernetzbaren Monomeren, das mindestens zwei mit ionisierender Strahlung reaktionsfähige Ethylenfunktionen aufweist, wobei das vernetzte Polyvinylalkohol-Hydrogel zu 70 bis 95 Gew.-% aus von Polyvinylalkohol abgeleiteten Ketten und zu 5 bis 30 Gew.-% aus von den vernetzenden Monomeren abgeleiteten Ketten besteht und mindestens ein Teil der OH-Gruppen des Polyvinylalkohol-Hydrogels durch $-OSO_3H$-Gruppen ersetzt ist, die mindestens 15 Gew.-% des Trägers ausmachen.

2. Träger nach Anspruch 1, dadurch gekennzeichnet, daß die vernetzenden Monomeren ausgewählt werden aus Ethylenglycoldiacrylat, Triethylenglycoldiacrylat, Tetraethylenglycoldiacrylat oder -triacrylat, Trimethylolpropantriacrylat, Ethylenglycoldimethacrylat, Triethylenglycoldimethacrylat, Tetraethylenglycoldimethacrylat, Trimethylolpropantrimethacrylat, Pentaerythrittetraacrylat, Pentaerythrittetramethacrylat und Mischungen davon.

3. Fester Träger nach Anspruch 1, dadurch gekennzeichnet, daß es sich bei den vernetzenden Monomeren handelt um Triethylenglycoldiacrylat und Tetraethylenglycoldiacrylat.

4. Fester Träger nach einem der Ansprüche 1 bis 3, dadurch gekennzeichnet, daß er in Form eines Pulvers mit einer Korngrösse von 50 bis 100 µm vorliegt.

5. Verfahren zur Herstellung eines festen Trägers nach einem der Ansprüche 1 bis 4, dadurch gekennzeichnet, daß es die folgenden Stufen umfaßt:

a) Vernetzen des Polyvinylalkohols, indem man mit ionisierender Strahlung bestrahlt eine Mischung aus 70 bis 95 Gew.-% Polyvinylalkohol und 5 bis 30 Gew.-% mindestens eines vernetzenden Monomeren in einer sauerstofffreien Atmosphäre und

b) Durchführung einer Chlorsulfonierung mit dem so erhaltenen venetzten Polyvinylalkohol, um mindestens einen Teil der OH-Gruppen des Polyvinylalkohols durch $-OSO_3H$-Gruppen zu ersetzen zur Herstellung eines Trägers, der mindestens 15 Gew.-% $-OSO_3H$-Gruppen aufweist.

6. Verfahren nach Anspruch 5, dadurch gekennzeichnet, daß in der Stufe (a) der Polyvinylalkohol in wäßriger Lösung vorliegt.

7. Verfahren nach einem der Ansprüche 5 und 6, dadurch gekennzeichnet, daß die Polyvinylalkoholkonzentration der wäßrigen Lösung 25 bis 30 Gew.-% beträgt.

8. Verfahren nach einem der Ansprüche 5 bis 7, dadurch gekennzeichnet, daß der als Ausgangsmaterial verwendete Polyvinylalkohol in Form einer 4%-igen wäßrigen Lösung mit einer Viskosität von 3,5 bis 5 mPa•s vorliegt.

9. Verfahren nach einem der Ansprüche 5 bis 8, dadurch gekennzeichnet, daß die Bestrahlung mit Gamma-Strahlung in einer Gesamt-Strahlungsdosis von 8 bis 16 Mrad durchgeführt wird.

10. Verfahren nach einem der Ansprüche 5 bis 9, dadurch gekennzeichnet, daß die Chlorsulfonierungsbehandlung darin besteht, einen Komplex aus Chlorsulfonsäure und Pyridin herzustellen und dann diesen Komplex mit dem vernetzten Polyvinylalkohol in Kontakt zu bringen.

11. Verfahren zur Abtrennung der in einer Flüssigkeit vorhandenen Lipoproteine, dadurch gekennzeichnet, daß man

– die Flüssigkeit mit einem festen Träger nach einem der Ansprüche 1 bis 4 in Kontakt bringt und

– anschließend die Flüssigkeit von dem Träger, an dem die Lipoproteine adsorbiert worden sind, trennt.

12. Verfahren nach Anspruch 11, dadurch gekennzeichnet, daß es sich bei der Flüssigkeit um Blut handelt.

13. Verfahren nach Anspruch 11, dadurch gekennzeichnet, daß es sich bei der Flüssigkeit um Blutplasma handelt.

FIG. 1

FIG. 2

FIG.3

FIG.4